# EUROPEAN PATENT SPECIFICATION

(11) **EP 3 028 714 B1**
(45) Date of publication and mention of the grant of the patent: **29.09.2021**
(21) Application number: 14758073.2
(22) Date of filing: 01.08.2014
(51) Int. Cl.: A61K 39/00

(54) **DIVALENT VACCINE COMPOSITIONS AND THE USE THEREOF FOR TREATING TUMOURS**
BIVALENTE IMPFSTOFFZUSAMMENSETZUNGEN UND IHRE VERWENDUNG IN DER THERAPIE VON TUMOREN
COMPOSITIONS VACCINALES BIVALENTES ET LEUR UTILISATION POUR LA THERAPIE ANTITUMORALE

(30) Priority: 02.08.2013 CU 20130110
(43) Date of publication of application: 08.06.2016
(73) Proprietor: Centro de Inmunologia Molecular, La Habana 11600 (CU)
(72) Inventor: SÁNCHEZ RAMÍREZ, Belinda, 11300 La Habana (CU); YGLESIAS RIVERA, Arianna, 11000 La Habana (CU); GUTIÉRREZ PÉREZ, Amelia, 10200 La Habana (CU); GONZÁLEZ SUÁREZ, Narjara, 17100 La Habana (CU)
(74) Representative: V.O.
(86) International application number: PCT/CU2014/000004
(87) International publication number: WO 2015/014327

(56) References cited:
- WO-A2-02/45746
- None

## Description

### TECHNICAL FIELD OF THE INVENTION

The present invention relates to biotechnology and immunology applied to human health. It particularly relates to a vaccine formulation for the treatment of malignant tumors.

### BACKGROUND OF THE INVENTION

Her1 and Her2 receptors are transmembrane glycoproteins with tyrosine kinase activity that belongs to a family of receptors known as the ErbB family (Normanno N, et al (2005) Curr Drug Targets 6, (3): 243-257). Her1 is overexpressed in lung, breast, head and neck, colorectal, pancreas, bladder, ovary tumors, glioblastomas (TM Brand, et al, (2011) Cancer Biol Ther 11 (9): 777-792); (Zhau HY et al, (1996) Proc Natl Acad Sci U S A 93, (26): 15152-15157; Liu XH, et al, (1993) J Clin Endocrinol Metab 77 (6): 1472-1478; Neal DE, et al, (1985) Lancet 1 (8425): 366-368; Gullick WJ, et al, Cancer Res 46 (1): 285-292; Salomon DS, et al, (1995) Crit Rev Oncol Hematol 19 (3): 183-232). Her1 overexpression is associated with poor prognosis in head and neck tumors and lung cancer, with a high risk of disease recurrence (Turkeri LN et al, (1998) Urology 51 (4): 645-649; Chow NH, et al, (1997) Anticancer Res 17 (2B): 1293-1296) and with decreased survival of patients with ovarian, colon, bladder, thyroid and head and neck cancer (Grandis et al, (1998) J Cell Biochemr 69 (1):55-62).

Furthermore, Her1 expression levels correlate with resistance to conventional therapies (Holbro T, et al, (2003) Exp Cell Res 284 (1): 99-110). Also, an aberrant Her2 receptor expression as compared with expression in normal tissues has been found in breast gastric, ovarian and prostate tumors (Tai W et al, (2010) J Control Release 146 (3): 264-275). In addition, deregulation of this receptor has been observed in the malignant transformation of the respiratory tract (Andrade Filho et al., 2010) and is a mechanism of tumor resistance to anti-Her1 therapies (Brand et al, (2011) Cancer Biol Ther 11 (9): 777-792). Her2 overexpression in breast tumors has been correlated with lower overall survival and relapse-free survival.

Many of the aforementioned tumors co-express high levels of Her1 and Her2, and the overexpression of both receptors is associated with decreased patient survival (Wiseman SM, et al, (2005) Cancer. May 1;103(9): 1770-7). Patients with Her2 positive breast tumors have a high risk of mortality associated with the disease, and in addition, the co-expression of Her1 and Her2 in these tumors significantly increases this risk, which indicates that Her1 expression in these carcinomas has a synergistic effect on Her2 expression (Suo Z et al, (2002) J Pathol 196 (1): 17-25). It has been experimentally demonstrated that there is synergistic interaction between these receptors in cell malignant transformation (Kokai Y et al, (1989) Cell 58 (2): 287-292). Other examples of tumors in which co-expression of Her1 and Her2 has been found are brain, ovarian, head and neck, lung, esophagus and colon cancers (Emanuel SL et al, (2008) Mol Pharmacol 73 (2): 338-348; Ako E et al, (2007). Oncol Rep 17 (4): 887-893; Venkateswarlu S et al, (2002) Oncogene 21 (1): 78-86).

It is well documented in the literature the physiological role of the aberrant expression and co-expression of Her1 and Her2 receptors in tumors. These receptors, through the formation of dimers and heterodimers (Pinkas-Kramarski R et al, (1996) EMBO J 15 (10): 2452-2467), regulate important processes in tumor biology, such as sustained proliferation, angiogenesis, inhibition of apoptosis, metabolic reprogramming and invasive and metastatic capacity (Hanahan D et al (2011) Cell 144 (5): 646-674).

Both receptors Her1 and Her2 have a similar structure consisting of an extracellular domain (ECD), a single transmembrane domain and an intracellular domain with tyrosine kinase activity. Her1 ECD (ECD-Her1) and Her2 ECD (ECD-Her2) have a molecular weight of 105 and 110 kDa respectively, and might have a different conformation in space than that of the rest of the molecule. The ECDs comprise four subdomains named I, II, III and IV. Subdomains I and III are the regions that form the ligand-binding site and contain potential glycosylation sites. Subdomain II is the critical region for dimerization between receptors, and it is called "dimerization arm" (Garrett TP et al, (2002) Cell 110 (6): 763-773; Ferguson KM et al, (2003) Mol Cell 11 (2): 507-517).

Seven Her1 ligands have been described, among them the most relevant for tumor formation are the Epidermal Growth Factor (EGF), that promotes the proliferation of epidermal cells which express the receptor, the Transforming Growth Factor (TGF) and Amphiregulin (Normanno et al, (2005) Curr Drug Targets 6 (3): 243-257). The ECD-Her1 is in dynamic equilibrium and has multiple conformations that include "closed" conformations and conformations where subdomain II is in a more flexible state. The binding of the ligand shifts the equilibrium towards "extended" or active conformation, a conformational state that is competent for dimerization (Ferguson KM, (2008) Annu Rev Biophys. 37: 353-373).

However, no specific ligand for Her2, has not been found. Signaling through this receptor does not require growth factors, since the receptor has an active or "extended" conformation of constitutive form which exposes its dimerization subdomain (Cho HS et al, (2003) Nature 421 (6924): 756-760). For this reason Her2 receptor is the preferred dimerization partner for all the other members of the ErbB family, including Her1 (Franklin MC et al, (2004) Cancer Cell 5 (4): 317-328). Heterodimerization with Her2 contributes to a decrease in the degree of endocytosis of Her1 and an increase of recycling to the membrane of the receptors that form the heterodimer (Lenferink A E et al, (1998) EMBO J 17 (12): 3385-3397).

As described by Baselga J et al, (2009) Nat Rev Cancer 9 (7): 463-475), the ligand-induced dimerization allows the autophosphorylation and transphosphorylation of tyrosine residues of the cytoplasmic region of the receptors. The phosphotyrosine residues generated, initiate multiple intracellular signaling pathways. One of the signaling pathways initiated is that of the mitogen activated protein kinases (MAPKs), in whose activation cascade participate the extra-cellular regulated protein kinases Erk1 and Erk2. They induce the expression of transcription factors that increase the transcription of genes involved in cell proliferation, such as cyclin D1 which promotes cell cycle progression to the G1/S phase. The heterodimeric combinations are the most potent signaling complexes and have direct control of the cell cycle (Pinkas-Kramarski R et al, (1996) EMBO J 15 (10): 2452-2467).

These tumor targets have been extensively validated in clinical studies. The clinical effect of treatment with tyrosine kinase inhibitors (TKI) of these receptors, such as gefitinib and lapatinib, has been evaluated (Ciardiello F et al, (2000) Clin Cancer Res 6 (5): 2053-2063; Kondo N et al, (2010) Oncol Rep 23 (4): 957-963). There are also reports from clinical trials that using monoclonal antibodies (MAbs) against the aforementioned targets, such as cetuximab (Jean GW et al, (2008) Pharmacotherapy 8(6):742-54) and nimotuzumab (Ramos TC, et al, (2006) Cancer Biol Ther 5 (4): 375-379), that are specific for the ECD of Her1, and trastuzumab, that recognizes Her2 ECD (Clifford A, (2007) N Engl J Med; 357:39-51). Despite the potent antitumor effect of some of these therapies, such as cetuximab, molecular mechanisms of resistance have been described, among them increased levels of expression of another receptor of the family, for instance Her2 (Brand TM et al, (2011) Cancer Biol Ther 11 (9): 777-792). On the other hand, a better response to pertuzumab anti-Her1 murine MAb in breast and lung carcinomas expressing Her2 heterodimers has been found with other members of the ErbB family (Agus DB et al, (2005) J Clin Oncol 23 (11): 2534-2543). This supports the idea that inhibition of heterodimerization is an attractive strategy for cancer therapy.

Furthermore, active therapy with cancer vaccine has emerged as a new therapeutic option whose aim is to activate the immune system response in patients with tumors that express Her1 and Her2. This type of therapy, however, is challenging, since it involves stimulation of the immune system depressed by the suppressive effect of the tumor, which has made it necessary for vaccine formulations to use adjuvants in order to help generate an effective response. Such is the case of the adjuvant of very small size proteoliposomes (VSSP) derived from the outer membrane proteins of *Neisseria meningitidis,* which is capable of activating dendritic cells (DC) and polarizing the immune response towards the pattern of T-Helper Type 1 Response (Th1) as described in WO 02/45746 patent published on 13.12.2002.

Her1 and Her2 receptors have been targets of different vaccine candidates evaluated in preclinical and clinical studies. In the design of vaccines based on Her2, DCs vaccines (Czerniecki BJ et al, (2007) Cancer Res 67 (4): 1842-1852), Her2 peptides and ECD-Her2 have been used. (Ladjemi MZ et al, (2010) Cancer Immunol Immunother 59 (9): 1295-1312). In phase II clinical studies with breast cancer patients, active immunization with peptide p369-377 (peptide E75) derived from the Her2 ECD and presented by MHC-I, using colony stimulating factor granulocyte-macrophage (GM-CSF) as adjuvant was evaluated. This vaccine is able to generate specific Citotoxic T Lymphocytes (CTL) *in vivo* against cells overexpressing Her2 and is currently in Phase II clinical studies (Knutson KL et al, (2002) Clin Cancer Res 8 (5): 1014-1018; Patil R et al, (2010) J Am Coll Surg 210 (2): 140-147). In addition, a therapeutic vaccine based on the ECD of Her2 and adjuvated with GM-CSF has been designed, the vaccine is capable of retarding the growth of carcinomas that express Her2 and prolong immunized animals survival (Helguera et al, (2006) Vaccine 16;24(3):304-16).

Vaccine compositions that stimulate or increase the antibody response against gangliosides and comprise an immunogen and an immunological adjuvant are described in patent application EP-A-661061 and US-A-6149921 patent. The immunogens described are VSSP formed by the association of the GM3 gangliosides N-acetylated (N-AcGM3) and N-GlicolilGM3 (N-GcGM3), with the outer membrane protein complex (OMPC) of bacterium *Neisseria meningitidis.* Immunogens N-AcGM3/VSSP and N-GcGM3/VSSP are of very small size, practically invisible to electronic microscope, soluble in water and with high buoyancy.

Patent WO-A-02145746 describes vaccine compositions that contain: (A) one or more poorly immunogenic antigens; (B) VSSPs with gangliosides incorporated, mainly N-AcGM3/VSSP and N-GcGM3/VSSP; and (C) optionally one or more adjuvants. Particularly a vaccine whose active ingredient is the ECD-Her1 and that uses VSSP and Montanide ISA51 VG as adjuvants is described. Preclinical studies with this vaccine demonstrated its antitumor effect *in vitro* and *in vivo* (Ramirez BS, et al, (2006) Int J Cancer 119 (9): 2190-2199; Ramirez BS, et al (2008) Vaccine 26 (38): 4918-4926) .

Although results have been obtained with monovalent therapies, they have not been sufficiently effective because since the therapies cannot completely remove the tumor mass and even when the tumor does decrease in size eventually it regresses after the emergence of resistant variants. This suggests that specific perturbations are not able to abrogate complex biological systems such as tumors. The explanation for this could be that resistant variants generated by monotherapies targeting members of the ErbB family, increase the expression levels of other members of the family (Brand TM, et al, (2011) Cancer Biol Ther 11 (9): 777-792) allowing tumors to escape the immune system attack. Due to this a multiple perturbation in the tumor biology through the inactivation of more than one ErbB receptor could result in increased efficacy.

Patent application WO-A-02145746 also claims a composition comprising growth factor receptors Her-1, Her-2 and PDGF-R or any of its variants containing the extracellular domain with or without the transmembrane region. However, neither this application nor any other document of the art teaches how to combine the two receptors, particularly the ECDs of Her1 and Her2 receptors to produce a Bivalent Vaccine effective in inducing the production of antibodies in the vaccinated individual.

The authors of the present invention have discovered that when the ECDs of Her1 and Her2 and VSSP-GM3 are mixed in the same proportion a vaccine composition able of inducing antibody titers production that inhibit phosphorylation of Her1 and Her2 is obtained and these antibodies have an anti-proliferative effect on tumor cells. The inventors also found that the proportion in which the ECDs of Her1 and Her2 are combined is critical to obtain the desired immune response, that is, that not any combination of the ECDs of Her1 and Her2 receptors triggers this immune response. The object of the present invention is new vaccine compositions with the same proportion of the ECDs of Her1 and Her2 receptors and VSSPs-GM3 for subcutaneous administration.

A further object of the present disclosure is the vaccine for use mentioned above, for use in a method for treating patients suffering from a disease caused by tumors that express the receptors of growth factors Her1 and Her2 comprising the subcutaneous administration of said vaccine.

A further object of the present disclosure not part of the invention is vaccine compositions with the same proportion of the ECDs of Her1 and Her2 receptors and VSSP-GM3 and an additional adjuvant which may be oily or not oily.

### DETAILED DESCRIPTION OF THE INVENTION:

The present invention provides a vaccine composition for use according to claim 1.

The present disclosure relates to a vaccine composition comprising the ECDs of Her1 and Her2 receptors or fragments thereof used to induce an immune response against malignant tumors that express Her1 and Her2 receptors. This vaccine composition further comprises very small size proteoliposomes purified from proteins of the outer membrane of *Neisseria meningitidis* and the GM3 (VSSP-GM3). In a particular embodiment the composition of the present invention further comprises a pharmaceutically suitable adjuvant. These pharmaceutically suitable adjuvants include Alumina.

Surprisingly the inventors of the present invention found that the proportion in which ECDs of the receptors are combined is relevant in the induction of the immune response against malignant tumors that express Her1 and Her2 receptors. The bivalent vaccine composition described herein comprises the ECDs of Her1 and Her2 receptors in the same proportion. The mixture of both active substances that is the ECDs of Her1 and Her2 receptors in other proportions does not have the desired effect on the immune system. In one embodiment not covered by the claimed invention the composition comprises the ECDs of Her1 and Her2 receptors in a concentration range from approximately 100 to approximately 800 ug/dose. More particularly the composition comprises 800 ug/dose of either the ECDs of Her1 and Her2 receptors or fragments thereof and additionally comprises 1.2 mg/dose of VSSP-GM3, administered subcutaneously in an aqueous solution.

In another embodiment not covered by the claimed invention, the composition comprises these doses of 800 ug/dose of each of the ECDs of Her1 and Her2 receptors or fragments thereof and 1.2 mg/dose of VSSP-GM3 that can be mixed with an adjuvant which can be Montanide ISA 51 or Alumina.

Another embodiment not covered by the claimed invention relates to a vaccine composition comprising the ECDs of Her1 and Her2 receptors or fragments thereof to be used in the induction of an immune response as a method for the treatment of malignant tumors that express Her1 and Her2 receptors. Said method comprises the subcutaneous administration of at least one dose of the ECDs of Her1 and Her2 receptors or fragments thereof in a dose range from approximately 100 to approximately 800ug/dose and additionally of 200 ug/dose at approximately 1.2 mg/dose of VSSPs-GM3. The administration may be by subcutaneous injection every two weeks to complete a total of 5 doses and then a maintenance dose will be administered for 1 year.

The ECDs of Her1 and Her2 receptors or fragments thereof are obtained by protein synthesis or gene engineering techniques

### BRIEF DESCRIPTION OF THE FIGURES:

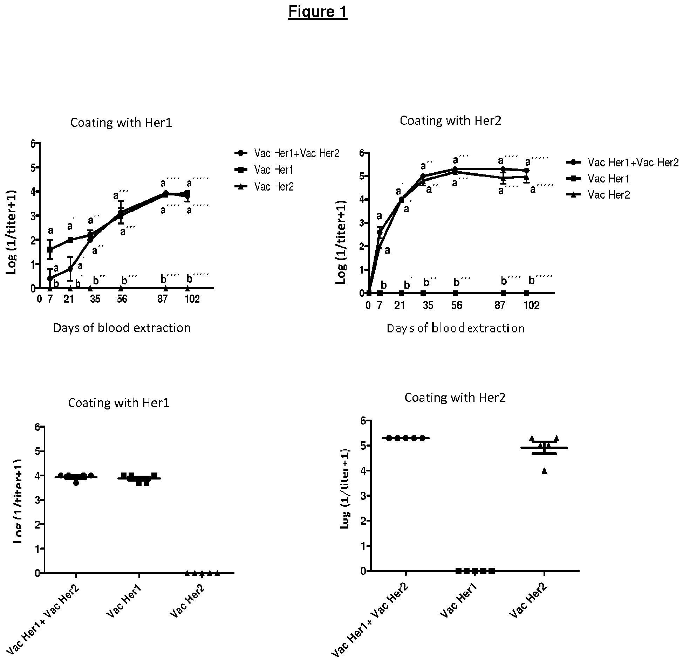

**Figure 1** shows the kinetics of IgG Abs in BALB/c mice (n = 5) immunized subcutaneously four times every 14 days, and 30 days after the last immunization with 100 µg of ECD-Her1 and 100 µg of ECD-Her2 in 200µg of VSSPs. Blood draws were performed on days 7, 21, 35, 56, 87 and 102 to measure IgG Abs titers in the immune sera using ELISA assay. Abs IgG kinetics are expressed as the Log (1/titre media+1) for each extraction day, when it was coated with the ECD-Her1 (a) and ECD-Her2 (b). Abs response of individual mice (n = 5) is shown in the figure, the response is specific to ECD-HER1 (c) and ECD-Her2 (d) and was measured by plotting the reciprocal logarithm of the Abs title plus 1, on day 87 of the immunization protocol. Different letters indicate statistical significance as tested by two-way ANOVA (treatment group factor), using Bonferroni test correction (p <0.05).

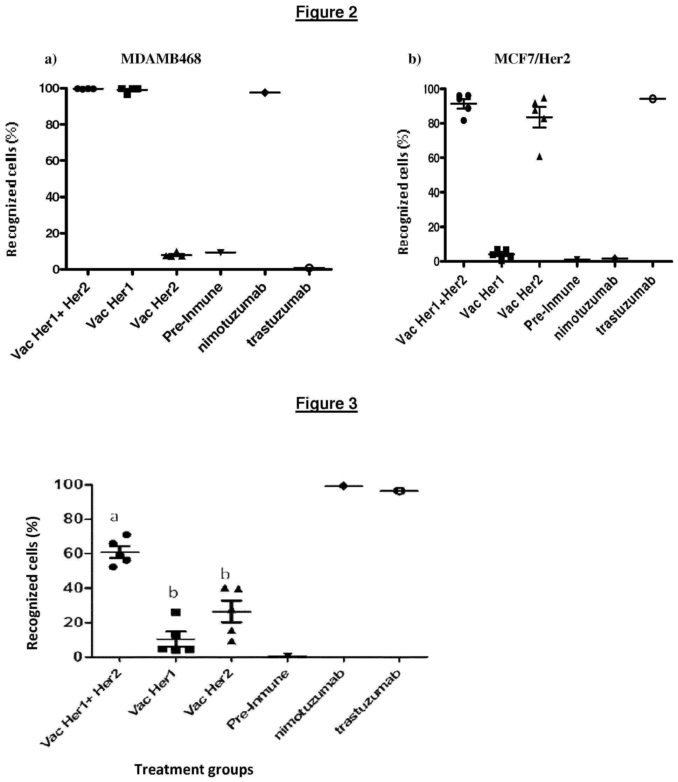

**Figure 2** shows the recognition of MDAMB468 and MCF7/HER2 tumor cell lines by immune sera induced by Bivalent Vaccine Her1+Her2 and monovalent vaccines Her1 and Her2, using flow cytometry. The aforementioned human tumor cell lines were incubated with the immune sera (n=5) or with a mixture of pre-immune sera diluted 1:200, in day 35 of the immunization protocol, as well as the controls of the expression of receptors: MAbs nimotuzumab and trastuzumab, at a concentration of 10µg/ml. Subsequently, the cells were labeled with goat PAbs anti-mouse IgG conjugated with fluorescein isothiocyanate. The labeled cells were visualized by flow cytometry. The percentage of cells recognized by the sera and the MAbs was determined using Flow Jo software version 5.7.2. Different letters indicate statistical significance as tested by two-way ANOVA (treatment group factor), using Bonferroni test correction (p <0.05).
**Figure 3** shows the recognition of H292 tumor cell line by immune sera induced by Bivalent Vaccine Her1+Her2 and monovalent vaccines Her1 and Her2, using flow cytometry. Human tumor cells were incubated on day 35 of the immunization protocol with immune sera (n = 5) or with a mixture of pre -immune sera diluted 1:300, as well as the controls of the expression of receptors: MAbs nimotuzumab and trastuzumab at concentration of 10µg/ml. Subsequently, the cells were labeled with goat PAbS anti-mouse IgG conjugated with fluorescein isothiocyanate. The labeled cells were visualized by flow cytometry. The percentage of cells recognized by the sera and MAbs was determined using Flow Jo software version 5.7.2. Different letters indicate statistical significance as tested by two-way ANOVA (treatment group factor), using Bonferroni test correction (p <0.05).

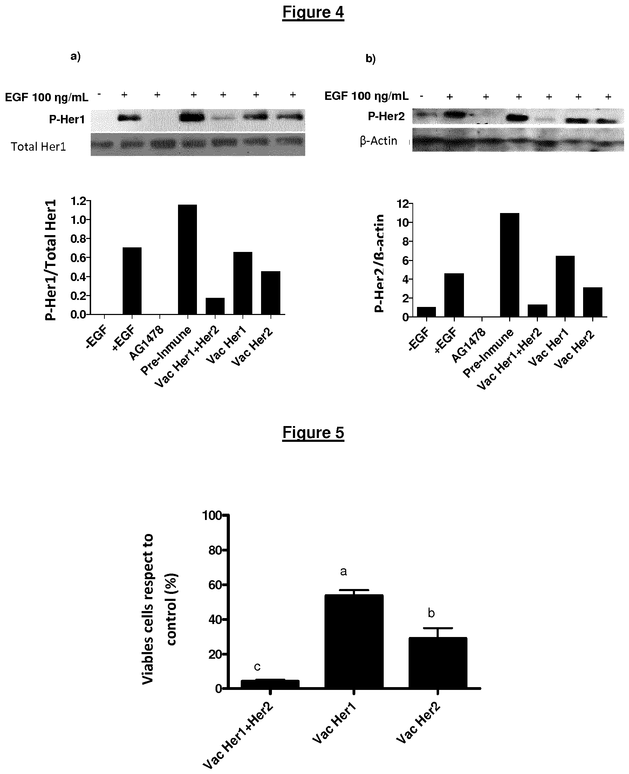

**Figure 4** shows the inhibition of Her1 and Her2 phosphorylation caused by the immune sera induced by Bivalent Vaccine Her1+Her2 administration. H292 cells were incubated in a mixture of five immune sera induced by Bivalent Vaccine Her1+Her2 and Monovalent Vaccines Her1 and Her2. Levels of Her1 and Her2 phosphorylation after stimulation with EGF and with the different treatments were determined by Western blot. Untreated cells were used as negative control and the treatment with a mixture of pre-immune sera (PI) as a negative control of specificity. TKI AG1478 was used as positive control for inhibition. The pictures show the levels of P-Her1 (a) and P-Her2 (b) obtained with different treatments. Bar graphs show the densitometric analysis of the pictures from one representative experiment chosen from the two experiments performed.
**Figure 5** shows the anti-proliferative effect of the Abs induced by Her1+Her2 bivalent vaccine on H292 tumor line. H292 cells were incubated for 48 hours in a mixture of five immune sera, diluted 1:20, on day 87 of the immunization protocol. The mixture of PI sera was used as negative control in the experiment. Cell viability was determined by the MTT colorimetric method, determining the difference of optical density (OD) by subtracting OD at 540 to the value of OD at 620 nm. The maximum viability percentage was found when subtracting the difference of absorbance of the incubation with PI serum. Bars represent the mean of triplicates of three experiments for each treatment. Different letters indicate statistical significance according to Dunnett T3 test for a vs b, p < 0.01 for a vs c, p < 0.001.

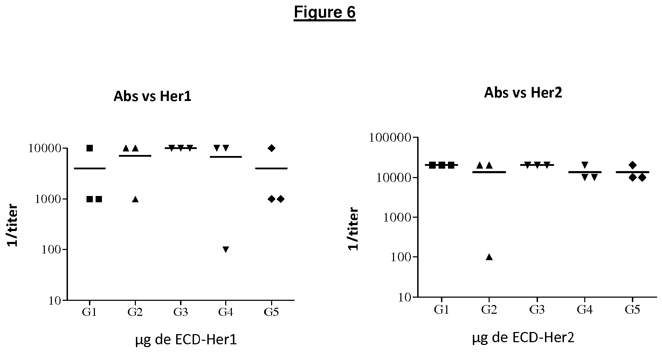

**Figure 6** shows the titers of Abs that recognize ECD-Her1 and ECD-Her2 induced by Bivalent Vaccine Her1+ Her2 formulations that contain equal or not equal amounts of each receptor. BALB/c mice (n=5) were immunized subcutaneously four times every 14 days with: Group 1, 100 µg of ECD- Her1 and 100 µg of ECD- Her2 ; Group 2, 100 µg of ECD- Her1 and 200 µg of ECD- Her2 ; Group 3, 100 µg of ECD- Her1 and 300 µg of ECD- Her2 ; Group 4, 200 µg of the ECD-Her1 and 100 µg ECD-Her2 ; Group 5, 300 µg ECD- Her1 and 100 µg ECD- Her2 . All formulations contained 200µg of VSSP. The determination of ECD-Her1 and ECD-Her2 specific antibody titers was performed on the serum by means of an ELISA assay, from a blood draw taken on day 56. Abs IgG kinetics are expressed as the media of log reciprocal plus one for each extraction day, when it was coated with the ECD-Her1 (a) and ECD-Her2 (b). IgG Abs response of individual mice (n=5) was determined by plotting the inverse of the antibody titers.

No statistical differences as tested by two-way ANOVA (treatment group factor), using Bonferroni test correction (p <0.05) were observed.

### EXAMPLES:

### Example 1: Specific antibodies titles raised against ECD-Her1 and DEC-Her2 induced by Her1+Her2 Bivalent Vaccine

Balb/c mice were immunized subcutaneously with a bivalent vaccine formulation containing 100 µg of ECD-Her1 and 100 µg of ECD-Her2. A second group of mice was immunized with a monovalent vaccine formulation containing 100 µg of ECD- Her1, and a third group was immunized with a monovalent vaccine formulation containing 100 µg of ECD-Her2. The three vaccine formulations mentioned contained 200 µg of VSSP adjuvant. Immunizations were performed on days 0, 14, 28, 42 and 72, and blood was drawn for processing the serum at days -2, 21, 56, 87 and 102. Specific antibody titers against the ECDs of Her1 and Her2 in the serum were determined using ELISA method.

The mice immunized with Her1+Her2 Bivalent vaccine raised specific IgG isotype antibodies against the ECD-Her1 and the ECD-Her2. The antibody titers induced against each of these receptors did not differ from those induced by the respective monovalent vaccines. In the case of the antibody response against ECD-Her1, both the titers induced by Her1+Her2 bivalent vaccine and the monovalent vaccine reached 1/10.000 values. The response against ECD-Her2 induced both by the bivalent and the monovalent vaccine reached antibody titers values of 1/200.000 (Figure 1).

This result shows that mixing the two receptors in a single vaccine formulation does not affect the immunogenicity of each of them individually, which validates the potential use of this vaccine formulation.

### Example 2: Recognition of Her1⁺/Her2⁻, Her1⁻/Her2⁺ tumor lines by sera induced by Bivalent Vaccine Her1+Her2

The sera from mice immunized with the Bivalent Vaccine Her1+Her2 and Monovalent Vaccines Her1 and Her2 , diluted 1/200 were incubated with 10⁵ cells of different tumor cell lines: MDA-MB468 (ATCC,HTB-132), derived from breast adenocarcinoma, and MCF7/Her2, generated from the transfection of MCF7 (ATCC HTB-22) cell line with full-length Her2. The PI sera were used as negative specificity controls. The Mab nimotuzumab, that recognizes Her1, and Herceptin monoclonal antibody, that recognizes Her2, were used as positive controls.

The sera generated by the Bivalent Vaccine recognized the same percentage of MDA-MB468 cells as the sera generated by Monovalent Vaccine Her1. Furthermore, the sera generated by the Bivalent Vaccine recognized the same percentage of MCF7/Her2 cells as the sera generated by Monovalent Vaccine Her2 according to Dunnett's T3 (p> 0.05) test (Figure 2) . The intensity of recognition of these receptors was also very similar (Table 1).

This demonstrates that the antibodies induced the bivalent formulation containing the truncated Her1 and Her2 receptors does not affect the recognition of the full-size Her1 and Her2 receptors in the membrane of tumor cells. It also shows that the quality of this recognition is not affected when the antibodies against Her1 and Her2 are generated from a formulation based on both receptors, since the recognition was the same as that obtained with the sera from the monovalent vaccines both in number of recognized cells and the intensity of the recognition.

**Table1: Mean fluorescence intensity (MFI) average of MDAMB468 and MCF7/HER2 cells recognized by the immune sera generated by the treatments.**

| Treatment | MDAMB 468 | MCF7 Her2 |
|---|---|---|
| | MFI | MFI |
| Vac Her1·Her2 | 440.05 | 140.99 |
| Vac Her1 | 370.23 | 4.31 |
| Vac Her2 | 4.02 | 104.05 |

### Example 3: Recognition of Her1⁺/Her2⁺ tumor line by sera induced by Her1+Her2 Bivalent Vaccine

Sera from mice immunized with Her1+Her2 Bivalent Vaccine and monovalent vaccines Her1 and Her2, diluted 1/300 were incubated with tumor cell line H292 (ATCC CRL-1848), derived from squamous cell carcinoma of the lung. The pre-immune sera were used as negative specificity controls. MAb nimotuzumab and MAb Herceptin were used as positive controls.

The percentage of H292 cells recognized was statistically higher in the sera generated by the bivalent vaccine, as compared to the recognition of the sera generated by Her1 and Her2 monovalent vaccines, according to Dunnett T3 test (p < 0.05) (Figure 3). This demonstrates that the polyclonal antibodies induced by the bivalent formulation have a higher threshold of recognition of tumor cells due to its ability to simultaneously recognize both receptors, Her1 and Her2, on the membrane of cells. This suggests that the bivalent vaccine has a higher potential with respect to the monovalent vaccines, in terms of biological effect on H292 tumor cells, determined by the number of recognized receptors. Such is the case of the inhibition of activation of the receptors from the Her family, which are involved in the proliferation of tumors.

### Example 4: Inhibition of the activation of HER1 and HER2 receptors by bivalent Her1+ Her2 vaccine

Sera from mice immunized with Bivalent Vaccine Her1+Her2 and monovalent vaccines Her1 and Her2, diluted 1/100 were incubated with cells from H292 tumor cell line. The cells were stimulated with 100 ng/mL of EGF for 10 min and then lysed. The effect of immune sera on the inhibition of EGFR phosphorylation was determined by Western blotting assay, using specific antibodies for the detection of phosphorylated EGFR and total EGFR. In this assay AG1478 tyrosine kinase inhibitor at 10 µM was used as positive control of the phosphorylation inhibition, and PI serum was used as negative control of specificity.

Sera generated by the Bivalent Vaccine inhibited the activation of Her1 and Her2 receptors, measured in terms of phosphorylation, to a greater extent than sera of monovalent vaccines Her1 and Her2 . The films from western blotting were subjected to densitometric analysis. The data from the densitometric analysis yielded that the decrease in Her1 phosphorylation caused by the immune sera induced by Bivalent Vaccine Her1+Her2 with respect to PI serum was 11.2 times. When the same analysis was performed to the sera induced by monovalent vaccine Her1, the decrease of Her1 phosphorylation was 1.7 times. In the case of sera from the Monovalent Vaccine Her2 group the decrease was 2.2 times. In the case of Her2, the decrease of Her2 phosphorylation induced by sera from mice immunized with Her1+Her2 Bivalent Vaccine with respect to PI serum was 8.7 times. When the same analysis was performed to the sera induced by monovalent vaccine Her1, the decrease in Her2 phosphorylation was 1.7 times, and for the sera from the Monovalent Vaccine Her2 group the decrease was 3.5 times (Figure 4) .

This demonstrates the superiority of the Bivalent Vaccine with respect to the Monovalent Vaccines. The Bivalent Vaccine could not only be able to directly inhibit the phosphorylation of Her1 or Her2 receptors, that form homodimers (Her1/Her1 and Her2/Her2) through antibodies that block ligand binding and against the dimerization arm, but it could also be more efficient than the monovalent vaccines in reducing the activation of the receptors that are part of a Her1/Her2 heterodimer.

### Example 5: Anti-proliferative effect of the sera induced by Her1+Her2 Bivalent Vaccine on Her1+/Her2+ tumor line

H292 cells were incubated for 48 h with sera from mice immunized with Bivalent Vaccine Her1+Her2 and Monovalent Vaccines Her1 and Her2, diluted 1:20. After this time, the culture supernatant was removed and the MTT reagent was added at a concentration of 1mg/mL. After 4hrs of incubation, the supernatant was removed and 100 µL of dimethylsulfoxide were added to dissolve the formazan crystals; absorbance was measured determining the difference of OD by subtracting OD at 540 to the value of OD at 620 nm with an ELISA reader. Viable cells were evaluated by determining the difference in absorbance between 540nm and 620nm. The difference of absorbance obtained in the PI serum was taken as maximum viability percentage.

Tumor cells treated with the sera induced by Bivalent Vaccine Her1+Her2 had less viability than tumor cells incubated with the serum induced by the monovalent vaccines, according to Dunnett's T3 statistic test (p < 0.05 (Figure 5). This demonstrates that the administration of the Bivalent Vaccine induced a higher antitumor effect on cells, in terms of anti-proliferative effects, than the monovalent vaccines expressing Her1 and Her2 receptors. This suggests its potential to be an effective therapeutic agent against epithelial tumors expressing both receptors.

### Example 6: Non-equivalent doses of ECD-Her1 and ECD-Her2 decrease the capacity of the antibodies generated to recognize H125/Her1+/Her2+

Balb/c mice were immunized subcutaneously with a bivalent vaccine formulation containing non-equivalent combinations of the ECD-Her1 and ECD-Her2. Group 1 was immunized with 100 µg of ECD-Her1 and 100 µg ECD-Her2 ; group 2 with 100 µg of ECD-Her1 and 200 µg ECD-Her2 ; group 3 with 100 µg of ECD-Her1 and 300 µg of ECD-Her2 ; group 4 with 200 µg of ECD-Her1 and 100 µg of ECD-Her2 ; group 5 with 300 µg of ECD-Her1 and 100 µg ECD-Her2. All mentioned adjuvant vaccine formulations contained 200 µg of VSSP. Immunizations were given on days 0, 14 and 28 via subcutaneous injections and blood was drawn to process the serum on day 42. Specific antibody titers against the ECDs of Her1 and Her2 were determined using ELISA method and the capacity of the above-mentioned sera to recognize H125 tumor cell line, which expresses Her1 and Her2, was assessed by FACS (Figure 6).

The percentage of tumor cells recognized by the immune sera was 100% for all groups evaluated, which is explained by the ability of formulation variants evaluated to induce antibody titers against Her1 and Her2. However, the recognition quality, measured in terms
of mean fluorescence intensity (MFI) was higher for group 1, wherein equivalent doses of both receptors (Table 2) were combined.

**Table 2: Recognition of H125 tumor cell line by the immune sera induced by Her1+Her2 bivalent vaccine formulations containing equivalent and non-equivalent doses of each receptor.**

| **Groups** | G1 | G2 | G3 | G4 | G5 |
|---|---|---|---|---|---|
| **ECD-Her1 (µg)** | 100 | 100 | 100 | 200 | 300 |
| **ECD-Her2 (µg)** | 100 | 200 | 300 | 100 | 100 |
| **Recognized cells (%)** | 100 | 100 | 100 | 100 | 100 |
| **MFI** | 90,86 | 71,3 | 45,7 | 60,6 | 53,63 |

| | | | | | |
|---|---|---|---|---|---|
| MFI: mean fluorescence intensity | | | | | |

## Claims

1. A vaccine composition comprising the ECDs of Her1 and Her2 receptors, or immunogenic fragments thereof, in the same proportion and in a concentration range from approximately 100 to approximately 800 µg/dose, and very small size proteoliposomes derived from proteins of the outer membrane of *Neisseria meningitidis* and GM3 (VSSP-GM3), for use in inducing an immune response against malignant tumors that express Her1 and Her2 receptors; wherein the composition is for subcutaneous administration.

2. The vaccine composition for use according to claim 1 wherein said composition further comprises a pharmaceutically acceptable adjuvant.

3. The vaccine composition for use according to claim 2 wherein the pharmaceutically acceptable adjuvant is Montanide ISA 51.

4. The vaccine composition for use according to claim 2 wherein the pharmaceutically acceptable adjuvant is Alumina.

5. The vaccine composition for use according to claim 1, wherein the composition is for subcutaneous administration every two weeks to complete a total of 5 doses and subsequently monthly as maintenance dose for at least one year.

6. The vaccine composition for use according to claim 1, wherein the immune response against malignant tumors that express Her1 and Her2 receptors is an immune response inhibiting proliferation of said tumors.

## Patentansprüche

1. Impfstoffzusammensetzung, umfassend die ECDs von Her1- und Her2-Rezeptoren oder immunogene Fragmente davon im gleichen Verhältnis und in einem Konzentrationsbereich von etwa 100 bis etwa 800 µg/Dosis und sehr kleine Proteoliposome, abstammend von Proteinen der äußeren Membran von *Neisseria meningitidis* und GM3 (VSSP-GM3), zur Verwendung beim Induzieren einer Immunantwort gegen bösartige Tumore, die Her1- und Her2-Rezeptoren exprimieren; wobei die Zusammensetzung für subkutane Verabreichung ist.

2. Impfstoffzusammensetzung zur Verwendung nach Anspruch 1, wobei die Zusammensetzung ferner ein pharmazeutisch akzeptables Adjuvans umfasst.

3. Impfstoffzusammensetzung zur Verwendung nach Anspruch 2, wobei das pharmazeutisch akzeptable Adjuvans Montanid ISA 51 ist.

4. Impfstoffzusammensetzung zur Verwendung nach Anspruch 2, wobei das pharmazeutisch akzeptable Adjuvans Aluminiumoxid ist.

5. Impfstoffzusammensetzung zur Verwendung nach Anspruch 1, wobei die Zusammensetzung zur subkutanen Verabreichung alle zwei Wochen zur Vervollständigung von insgesamt 5 Dosen und anschließend monatlich als Erhaltungsdosis für wenigstens ein Jahr ist.

6. Impfstoffzusammensetzung zur Verwendung nach Anspruch 1, wobei die Immunantwort gegen bösartige Tumore, die Her1- und Her2-Rezeptoren exprimieren, eine Immunantwort ist, inhibierend die Proliferation der Tumore.

## Revendications

1. Composition vaccinale comprenant les ECD de récepteurs Her1 et Her2, ou des fragments immunogènes de ceux-ci, selon la même proportion et dans une plage de concentration d'approximativement 100 à approximativement 800 µg/dose, et des protéoliposomes de très petite taille dérivés de protéines de la membrane extérieure de *Neisseria meningitidis* et GM3 (VSSP-GM3), à utiliser pour induire une réponse immunitaire contre des tumeurs malignes qui expriment des récepteurs Her1 et Her2 ; dans laquelle la composition est destinée à une administration sous-cutanée.

2. Composition vaccinale à utiliser selon la revendication 1, dans laquelle ladite composition comprend en outre un adjuvant pharmaceutiquement acceptable.

3. Composition vaccinale à utiliser selon la revendication 2, dans laquelle l'adjuvant pharmaceutiquement acceptable est Montanide ISA 51.

4. Composition vaccinale à utiliser selon la revendication 2, dans laquelle l'adjuvant pharmaceutiquement acceptable est de l'alumine.

5. Composition vaccinale à utiliser selon la revendication 1, dans laquelle la composition est destinée à une administration sous-cutanée toutes les deux semaines pour terminer un total de 5 doses et ensuite mensuellement en tant que dose d'entretien pendant au moins un an.

6. Composition vaccinale à utiliser selon la revendication 1, dans laquelle la réponse immunitaire contre des tumeurs malignes qui expriment des récepteurs Her1 et Her2 est une réponse immunitaire inhibant une prolifération desdites tumeurs.
